# EUROPÄISCHE PATENTSCHRIFT

(11) **EP 0 947 163 B1**
(45) Veröffentlichungstag und Bekanntmachung des Hinweises auf die Patenterteilung: **13.12.2006**
(21) Anmeldenummer: 99810146.3
(22) Anmeldetag: 18.02.1999
(51) Int. Cl.: A61N 1/39, A61B 8/06

(54) **Vorrichtung zum Erzeugen eines Hilfssignals zum Bestimmen des Zeitpunkts einer Herzdefibrillation**
Device for generating an auxiliary signal for determining the time of a heart defibrillation
Dispositif pour produire un signal auxiliaire pour détecter l'instant d'une défibrillation cardiaque

(30) Priorität: 26.02.1998 EP 98810156; 10.06.1998 EP 98110668
(43) Veröffentlichungstag der Anmeldung: 06.10.1999
(73) Patentinhaber: Schiller, Alfred, 8914 Aeugst a.A. (CH); Stemple, Günter, 86916 Kaufering (DE)
(72) Erfinder: Schiller, Alfred, 8914 Aeugst a.A. (CH); Stemple, Günter, 86916 Kaufering (DE)
(74) Vertreter: Müller, Christoph Emanuel

(56) Entgegenhaltungen:
- EP-A- 0 465 241
- WO-A-91/16000
- WO-A-96/14014
- FR-A- 2 591 884
- PAULO CELSO BUDRI FREIRE ET AL: "COMPUTARIZED BLOOD-FLOW EVALUATION IN VASCULAR SURGERIES WITH A CW DOPPLER" CARDIOLOGY AND IMAGING, NEW ORLEANS, NOV. 4 - 7, 1988, Bd. 1, Nr. CONF. 10, 4. November 1988 (1988-11-04), Seite 225/226 XP000042053 HARRIS G;WALKER C

## Beschreibung

Die Erfindung betrifft eine Vorrichtung zum Erzeugen eines Hilfssignals mit den Merkmalen des Oberbegriffs des Anspruchs 1.

Es ist bekannt, bei Herzstillstand oder bei Herzstörungen Elektroschocks bzw. Herzmassagen einzusetzen. Defibrillatoren (zum Erzeugen von Elektroschocks) bzw. Herzmassagen werden häufig bei Erste-Hilfeleistungen eingesetzt. Elektrische Stimulationen können aber auch über externe Herzschrittmacher (external pacemakers) durchgeführt werden.

Es ist wichtig zu wissen, zu welchem Zeitpunkt und ob eine Defibrillation bzw. eine Herzmassage durchgeführt werden muss. Bei Tachycardien bzw. Rhytmusstörungen können Defribrillationen auch durchgeführt werden, wenn ein Puls spürbar, jedoch unregelmässig oder zu schnell ist. Eine Defibrillation sollte sonst aber nur durchgeführt werden, wenn beim Patienten kein Puls mehr festgestellt wird. Normalerweise wird dabei der (Carotis-) Puls des Patienten manuell getastet. Dabei kann es vor allem bei schwachem Puls zu einer Überlagerung des Pulses des Patienten mit dem Puls des Helfers kommen.

Ausserdem ist es wichtig, die Notwendigkeit einer Defibrillation bzw. einer Herzmassage sowie den Zeitpunkt, zu welchem mit der Reanimation aufgehört werden kann, zu bestimmen.

Es ist bereits bekannt, den Ablauf einer Reanimation mit einem Elektrokardiogramm zu überprüfen.

Eine solche Überprüfung der Reanimation weist aber verschiedene Nachteile auf. Die Durchblutung des Kopfes muss innerhalb von drei Minuten nach einem Herzstillstand wieder hergestellt werden, um irreparable Hirnschäden zu vermeiden. Während einer Reanimation kann zwar bereits ein Herzschlag einsetzen und zu einem entsprechenden EKG-Signal führen, während aber die Durchblutung des Hirns noch nicht ausreichend ist, um den Abbruch der Reanimation zu erlauben.

Allein aufgrund des EKG-Signals ist ausserdem die Notwendigkeit zum Auslösen des Defibrillationsstroms bzw. der Herzmassage schwierig zu ermitteln. Deshalb wird während der Reanimation der Puls des Patienten manuell gemessen. Eine Defibrillation wird nur bei Pulslosigkeit ausgelöst.

Defibrillation kann in verschiedenen Situationen nötig sein. Bei sogenanntem Kammerflimmern (d.h. bei unregelmässigen elektrischen Aktionspotentialen mit einer Frequenzbandbreite von 0 bis ca. 15 Hz) kann das Flimmern durch Defibrillation beendet werden. Nach erfolgter Defibrillation findet das Herz wieder seinen Eigenrythmus. Die Pumpleistung bei Kammerflimmern ist praktisch gleich null.

Bei der sogenannten Tachycardie ist der Pulsschlag bei nicht belastetem Herz sehr hoch. Das Schlagvolumen ist dabei erheblich reduziert. Tachycardien können zu Kammerflattern führen, dieses wiederum zu Kammerflimmern.

In beiden Fällen wird eine Defibrillation nur durchgeführt, sobald kein Puls mehr tastbar ist. Sobald Puls tastbar ist, kann synchron eine Defibrillation mit kleiner Energiestufe (Kardioversion) durchgeführt werden.

Bei Kammerflimmern erfolgt die Schockabgabe asynchron zum EKG, bei Kardioversion erfolgt die Schockabgabe synchron zur R-Zacke des EKG.

In EP 0 465 241 ist ein Diagnosegerät beschrieben, bei dem nach einer Arrythmiemessung die Notwendigkeit einer Defibrillation oder Herzmassage mit einem neuronalen Netzwerk bestimmt wird.

Das Gerät weist Mittel zum Erzeugen eines Hilfssignals auf, die mit einem Defibrillator oder mit einer Anordnung zum Ausführen einer Herzmassage koppelbar sind.

Bei allen Anwendungen von Defibrillationen wird die mechanische Tätigkeit des Herzens erfasst. Weil häufig in den Extremitäten des Patienten Blutleere herrscht, wird der Carotispuls an der Halsschlagader gemessen.

Vor allem bei Herz-Druckmassagen ist der mechanische Zustand des Herzens wichtig. Der Druckpunkt und die Massagefrequenz sollten in Abhängigkeit des gemessenen Pumpvolumens bestimmt und gegebenenfalls verändert d.h. optimiert werden.

Aus WO 91 16000 ist eine Anordnung zum Messen des Blutflusses bekannt. In WO 96 14014 wird ein Gerät beschrieben, mit dem unter anderem der Blutfluss in Blutgefässen bestimmt werden kann.

Es ist eine Aufgabe der vorliegenden Erfindung, die Nachteile des Bekannten zu vermeiden, insbesondere also eine Vorrichtung zum Erzeugen eines Hilfssignals als Entscheidungshilfe für das Auslösen einer Defibrillation und/oder einer Herzmassage bei Herzstörungen zu schaffen, welches sicherstellt, dass die Reanimation erst nach Erreichen einer ausreichenden Durchblutung des Hirns beendet wird.

Ausserdem soll die Notwendigkeit zum Auslösen der einzelnen Defibrilationsstromstösse bzw. Herzmassagen mit der erfindungsgemässen Vorrichtung festgestellt werden können.

Erfindungsgemäss werden diese Aufgaben mit einer Vorrichtung mit den Merkmalen des kennzeichnenden Teils des unabhängigen Patentanspruchs gelöst.

Die erfindungsgemässe Vorrichtung zum Erzeugen eines Hilfssignals zum Bestimmen des Zeitpunktes für das Auslösen einer Defibrillation und/oder einer Herzmassage bei Herzstörungen eines Säugetiers, insbesondere bei einem Menschen, besteht im wesentlichen aus einer Anordnung zum nicht-invasiven Messen des Blutflusses in den Kopf des Säugetieres und aus damit in Wirkverbindung stehenden Mitteln zum Erzeugen eines Hilfssignals. Die Mittel zum Erzeugen eines Hilfssignals sind mit einem Defibrillator oder mit einer Anordnung zum Ausführen einer Herzmassage koppelbar. Somit wird der Stromstoss für die Defibrillation bzw. die Herzmassage selbsttätig ausgelöst bzw. der Defibrillator blockiert, sobald ein ausreichender Blutfluss gemessen wird.

Vorteilhaft weist die Vorrichtung eine an sich bekannte Anordnung zum Erzeugen eines EKG-Signals auf.

Unter Herzstörungen wird in diesem Zusammenhang sowohl ein momentaner Herzstillstand als auch nicht ausreichender oder unregelmässiger Herzschlag verstanden. Unter Herzschlag wird hier und im folgenden die Kontraktion des Herzmuskels verstanden.

Das Auslösen von Elektroschocks zur Reanimation des Herzens (Defibrillation) und das Aufzeichnen von Elektrokardiogrammen während der Reanimation ist bereits bekannt als solches und nicht Gegenstand der vorliegenden Erfindung.

Weil das Hilfssignal in Abhängigkeit vom Wert des Blutflusses in den Kopf ausgelöst wird, ist gewährleistet, dass die Reanimation so lange fortgesetzt wird, bis eine ausreichende Durchblutung des Hirns sichergestellt ist. In Kombination mit einem EKG kann auch die Notwendigkeit einer Defibrillation besser beurteilt werden. Ausserdem führt die Abhängigkeit des Hilfssignals vom Wert des Blutflusses dazu, dass das Hilfssignal synchron mit dem Herzschlag erzeugt werden kann. Vor allem bei Herzmassagen erlaubt das Hilfssignal ein Feedback. In Abhängigkeit des Signals kann der Druckpunkt für die Massage optimiert werden. Das Hilfssignal bietet sich ausserdem auch zum Monitoring an.

Unter Blutfluss wird in diesem Zusammenhang das Blutvolumen verstanden, das pro bestimmte Zeiteinheit vom Herz in die Kopfpartie gepumpt wird. Der Blutfluss kann am einfachsten an der Halsschlagader gemessen werden.

Dank dem Hilfssignal kann ein Rettungssanitäter den Puls in der Halsschlagader verfolgen, ohne manuell messen zu müssen. In einem weitergehenden Ausführungsbeispiel wird der Wert des Blutflusses mit einem vorbestimmbaren Sollwert verglichen. Solange wenigstens der Wert der Amplitude des Blutflusses unter dem entsprechenden Sollwert liegt, wird das Hilfssignal ausgelöst. Sobald ein Herzschlag erkennbar ist, wird das Hilfssignal vorzugsweise zum Zeitpunkt des Herzschlages (d.h. bei maximalem Fluss) ausgelöst, solange der Wert des maximalen Flusses unter dem Sollwert liegt. Die Vergleichsanordnung errechnet ausgehend von den Signalen die Notwendigkeit und gegebenenfalls den Zeitpunkt zum Durchführen einer Reanimation.

Sobald nach einem Herzstillstand ein Herzschlag einsetzt, kann das Hilfssignal zur Überwachung der Effizienz der Herztätigkeit verwendet werden.

Als Hilfssignal ist grundsätzlich jedes Signal denkbar, welches der behandelnden Person den Blutfluss, bzw. den Herzschlag zum Auslösen der Defibrillation und/oder der Herzmassage anzeigt. Besonders einfach realisierbar und wirkungsvoll sind akustische oder optische Signale. Als optisches Signal ist beispielsweise eine auf einem Monitor angegebene Fluss-Kurve, aber auch eine Signallampe denkbar.

Das Hilfssignal kann kontinuierlich den Wert des Flusses als zeitabhängige Kurve anzeigen. Es ist aber auch denkbar, bei Erreichen der Amplitudenwerte ein Signal zu erzeugen.

Zum Messen des Blutflusses können grundsätzlich beliebige Flussmessanordnungen verwendet werden. Besonders vorteilhaft ist eine auf dem Dopplereffekt basierende Messzelle. Dopplermessungen zum Bestimmen der Geschwindigkeit des Blutes in Blutgefässen sind zu diagnostischen Zwecken bereits bekannt.

Es ist ausserdem auch denkbar, andere nicht auf einer Flussmessung basierende Pulsmessanordnungen zum Erzeugen des Hilfssignals zu verwenden.

Vorteilhaft bestehen die Mittel zum Erzeugen eines Hilfssignals aus einem akustischen oder einem optischen Signalgeber. Es ist aber auch denkbar, das Hilfssignal als elektronisches Signal direkt mit einem Defibrillator oder einer Anordnung zum Durchführen einer Herzmassage zu koppeln.

Als zusätzliche Massnahme ist es auch denkbar, die Elektrode des Defibrillators zum Bestimmen der mechanischen Herztätigkeit einzusetzen. Diese Elektrode wird während der Defibrillation zwischen dem Herz und dem Schlüsselbein des Patienten aufgelegt. Die Elektrode liegt dabei nahe der Herzspitze.

Die Detektion der Herztätigkeit kann dabei über Ultraschall erfolgen. Der Vorteil besteht darin, dass das Gel für die Defibrillation gleichzeitig für die Ultraschallmessung verwendet werden kann. Der Ultraschallsensor kann dabei direkt in eine Elektrode eingesetzt werden.

Das Leitgel wird also gleichzeitig für die Defibrillation und für die Ultraschallmessung verwendet, was die Applikation vereinfacht.

Als Gel wird vorteilhaft ein selbstklebendes Gel verwendet.

Weil bei einem Herzstillstand die Reanimation möglichst schnell begonnen werden muss, sollte keine Zeit verloren werden, um die Halsschlagader zum Aufsetzen der Anordnung zum Messen des Blutflusses zu lokalisieren. Gemäss einem besonders vorteilhaften Ausführungsbeispiel der vorliegenden Erfindung besteht die Anordnung zum nicht-invasiven Messen des Blutflusses aus einer Vielzahl von nebeneinander angeordneten Flussmesseinheiten. Die Signalausgänge der einzelnen Flussmesseinheiten sind mit einer Signalverarbeitungsanordnung gekoppelt. Dank dieser Ausführungsform kann die erfindungsgemässe Messanordnung auf den Hals des Patienten gelegt werden, ohne dass ein exaktes Aufsetzen nötig ist. Unabhängig davon, wie die Anordnung auf den Hals des Patienten aufgelegt wird, ist immer eine Flussmesseinheit richtig auf der Halsschlagader positioniert.

Vorteilhaft weist die Messanordnung etwa 3 bis 10 nebeneinander angeordnete Messeinheiten auf. Jede Messeinheit weist eine Oberfläche mit ca. 4 mm Durchmesser bis 8 mm Durchmesser auf. Als Messeinheiten werden bevorzugt auf der Messung des Dopplereffekts basierende piezoelektrische Messzellen eingesetzt. Es ist aber auch denkbar, andere bekannte Flussmesseinheiten in der erfindungsgemässen Art und Weise anzuordnen.
Vorteilhaft wird eine Reihe von nebeneinander liegenden Transducerelementen verwendet. Es können sowohl im gepulsten oder im continuous wave Verfahren arbeitende Transducer eingesetzt werden.

Alternativ ist es denkbar, zwei Reihen versetzt zueinander angeordnete Transducer vorzusehen. Damit kann die Wahrscheinlichkeit, dass in jedem Fall genau ein Transducer auf die Halsschlagader zu liegen kommt, auf nahezu 100 % vergrössert werden.

Die Erfindung wird im folgenden in Ausführungsbeispielen und anhand der Zeichnungen näher erläutert. Es zeigen:
- Figur 1: eine schematische Darstellung einer erfindungsgemässen Vorrichtung zum Erzeugen eines Hilfssignals,
- Figur 2: eine schematische Darstellung einer erfindungsgemässen Messanordnung,
- Figur 3: eine schematische Darstellung der erfindungsgemässen Vorrichtung in Gebrauch an einem menschlichen Körper,
- Figur 4: ein alternatives Ausführungsbeispiel einer Messanordnung, und
- Figuren 5a bis 5e: Ablaufschemen von verschiedenen Ausführungsbeispielen der vorliegenden Erfindung.

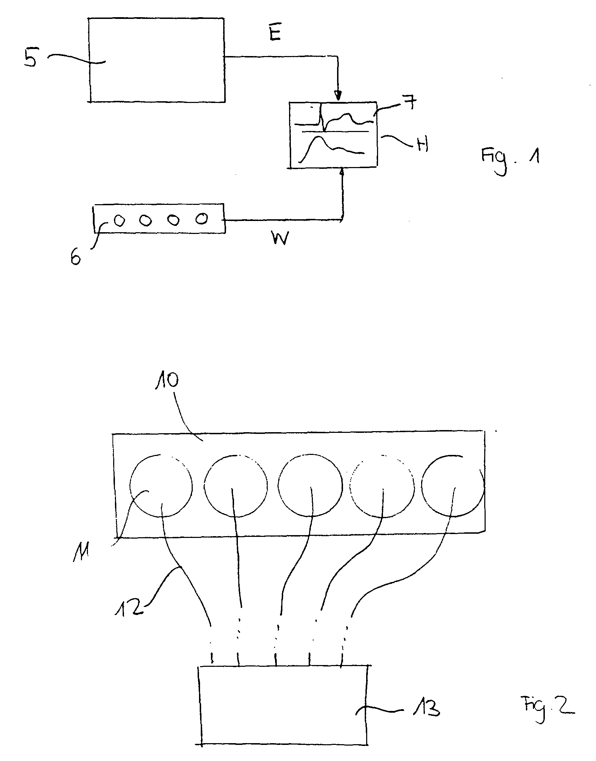

In Figur 1 ist schematisch eine erfindungsgemässe Vorrichtung 3 zum Erzeugen eines Hilfssignals H gezeigt. Die Vorrichtung 3 besteht im wesentlichen aus einer ersten Anordnung 5 zum Erzeugen eines EKG-Signals E und aus einer zweiten Anordnung 6 zum nicht-invasiven Messen des Wertes W eines Blutflusses B. Die Anordnungen 5, 6 sind mit den Mitteln 7 zum Erzeugen von Hilfssignalen H gekoppelt.

Die Mittel 7 zeigen eine EKG-Kurve und den Wert des gemessenen Blutflusses in Abhängigkeit der Zeit an.

Figur 2 zeigt schematisch eine erfindungsgemässe Messanordnung 10 zum nicht-invasiven Messen des Blutflusses. Die Messanordnung 10 besteht aus fünf nebeneinander angeordneten Flussmesseinheiten 11. Die Ausgänge 12 der Flussmesseinheiten 11 sind mit einer Signalverarbeitungsanordnung 13 gekoppelt. Die Signalverarbeitung 13 kann in der Vergleichsanordnung 8 (siehe Figur 1) integriert sein. Die Flussmesseinheiten 11 haben einen Durchmesser von 4 bis 5 mm. Es werden auf dem Prinzip der Dopplereffektmessung basierende Messeinheiten im gepulsten oder im continuous wave Verfahren verwendet. Je nach Einsatzzweck variert der Frequenzbereich der Transducer. Zum Messen des Carotis-Pulses wird eine Frequenz von 8 MHz verwendet. Zum Bestimmen des Pulses an tiefer liegenden Arterien können Frequenzen von 4 oder 2 MHz verwendet werden.

Gemäss vorliegendem Ausführungsbeispiel werden die Ausgänge 11 der Flussmesseinheiten mit einem Digitalen-Signal-Prozessor verbunden.

Wenn die erfindungsgemässe Messanordnung 10 auf den Hals eines Patienten gelegt wird, liegt immer eine der Flussmesseinheiten 11 auf oder benachbart zur Halsschlagader. Diese Flussmesseinheit 11 erzeugt ein Signal, welches den Wert des Blutflusses wiedergibt. Die übrigen Flussmesseinheiten, welche nicht direkt benachbart zu der Halsschlagader liegen, erzeugen ein schwächeres oder kein Signal.

Selbstverständlich ist es auch denkbar, mit einer Signalverarbeitungsanordnung 13 die Messwerte aller Flussmesseinheiten 11 zu berücksichtigen und ein integriertes Signal zu berechnen. Dies kann vor allem bei Anordnungen vorteilhaft sein, bei welchen die Dimensionen der Flussmesseinheiten 11 so gewählt sind, dass mehr als eine Flussmesseinheit 11 gleichzeitig benachbart zu der Halsschlagader liegt.

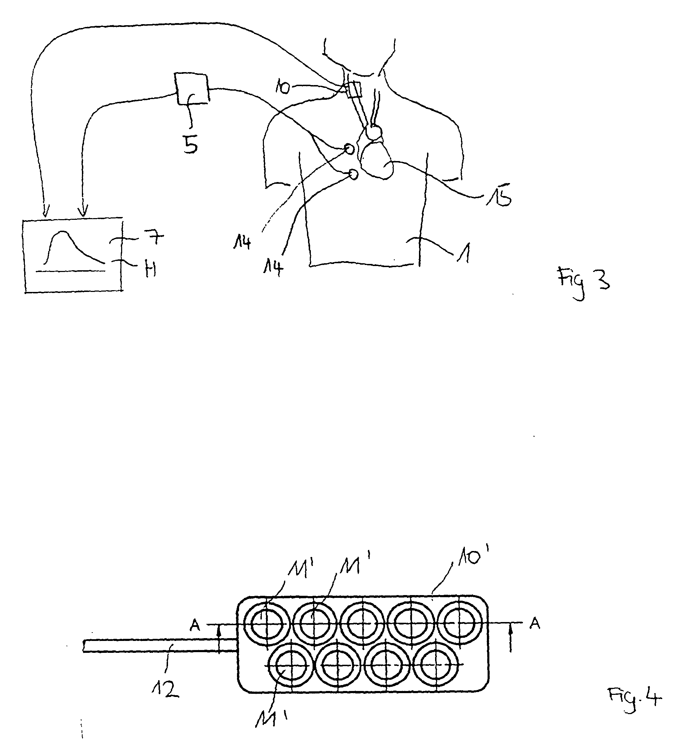

In Figur 3 ist schematisch ein menschlicher Körper 11 und die erfindungsgemässe Vorrichtung 3 gezeigt. Elektroden 14 werden zum Erzeugen eines Elektrokardiogramms auf dem Körper 1 positioniert. Eine EKG-Anordnung 5 erzeugt ein EKG-Signal E. Gleichzeitig wird eine zweite Anordnung 6 zum Messen des Blutflusses B mit einer Messanordnung 10 auf die Halspartie des Körpers 1 gelegt. Die Messanordnung 10 erzeugt ein Signal, das der Geschwindigkeit des Blutes entspricht und damit proportional zum Wert W des Blutflusses B ist.

Aufgrund des Hilfssignals H kann entschieden werden, ob ein Defibrillationsstoss oder eine Herzmassage ausgeführt werden soll und wie lange die Reanimation fortgesetzt werden soll. Die Reanimation soll solange durchgeführt werden, bis der Blutfluss physiologisch ausreichend für die Versorgung des Hirns ist.

In den schematischen Darstellungen ist das Hilfssignal H als optische Ausgabe auf einem Bildschirm dargestellt. Selbstverständlich sind die vorangehend beschrieben beliebige andere Hilfssignale denkbar.

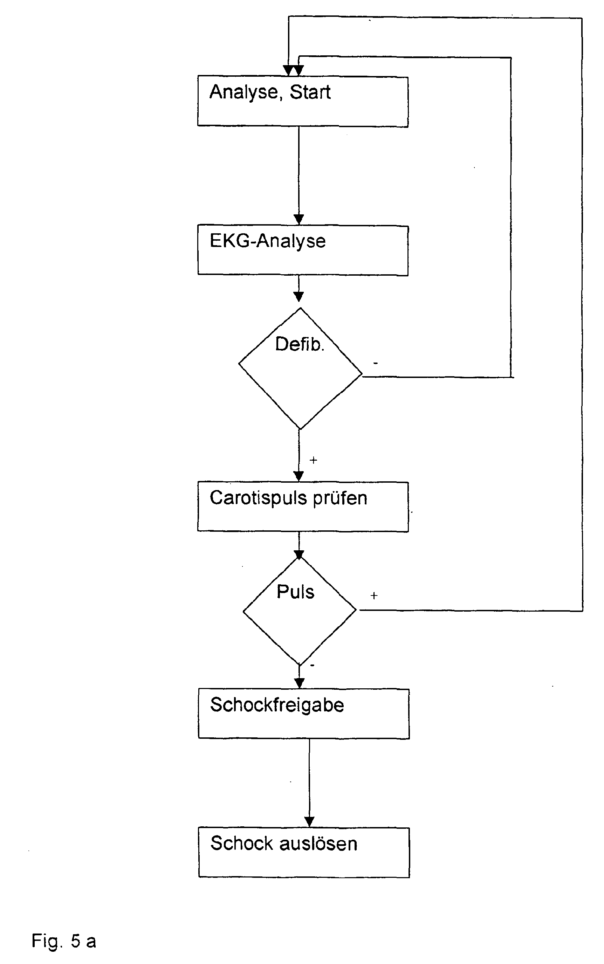

In Figur 4 ist ein alternatives Ausführungsbeispiel einer Messanordnung 10' gezeigt. Die einzelnen Flussmesseinheiten 11' sind in zwei Reihen versetzt zueinander angeordnet. Damit kann die Auflösung erhöht beziehungsweise die Wahrscheinlichkeit vergrössert werden, dass eine Flussmesseinheit jeweils genau auf der Schlagader liegt.

Die einzelnen Flussmesseinheiten 11, 11' können in Silikongummi eingegossen oder auch starr miteinander verbunden sein.

In Figur 5a ist schematisch der Ablauf eines ersten Ausführungsbeispiels für die semi-automatische Defibrillation gezeigt. Bevor eine Defibrillation durchgeführt wird, wird ein Analysezyklus durch Betätigung einer Starttaste durchgeführt. Dabei wird zuerst eine EKG-Analyse vorgenommen. Aufgrund der EKG-Analyse wird (automatisch) entschieden, ob eine Defibrillation notwendig ist. Wenn keine Defibrillation notwendig ist, wird der Analysezyklus wieder in den Anfangszustand zurückgesetzt.

Wenn aufgrund der EKG-Anlyse eine Defibrillation angezeigt ist, wird zuerst das erfindungsgemässe Hilfssignal H erzeugt, d.h. der Puls an der Carotis gemessen. Wenn Puls vorhanden ist, darf nicht defibrilliert werden. Wenn kein Puls vorhanden ist, wird ein Defibrillator zur Auslösung eines Schocks freigegeben. Die Auslösung des Schocks erfolgt dann manuell durch den Rettungssanitäter.

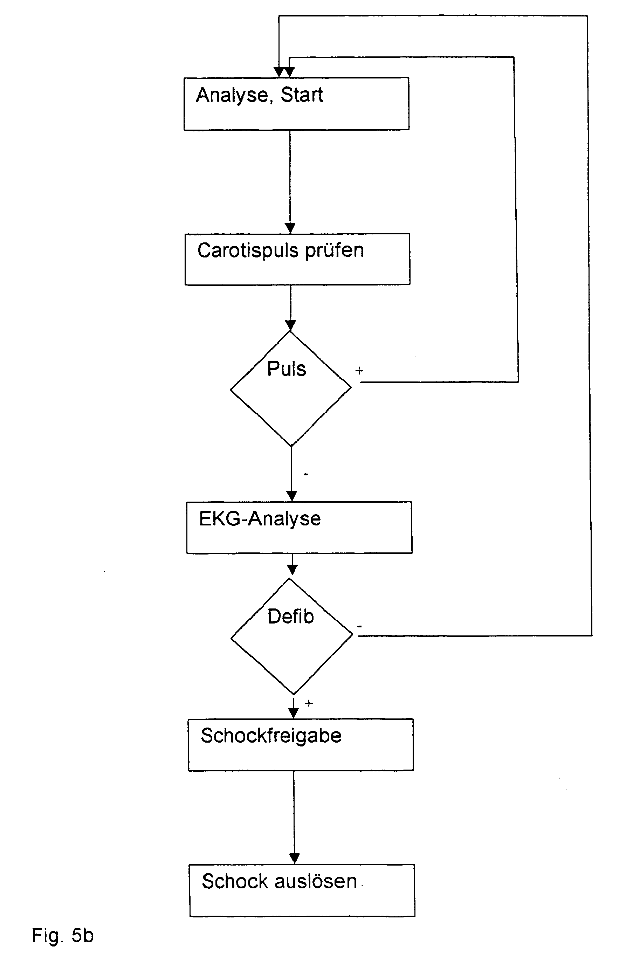

In Figur 5b ist ein alternatives Ausführungsbeispiel eines solchen Analysezyklus gezeigt. Dabei wird zuerst der Carotis-Puls geprüft. Wenn Puls detektierbar ist, ist keine Defibrillation nötig und der Analysezyklus wird in den Ursprungszustand zurückgesetzt. Eine EKG-Analyse wird bei diesem Ausführungsbeispiel nur durchgeführt, wenn nicht ausreichender Puls bzw. Fluss gemessen wird.

Die Entscheidung, ob ausgehend von einem gemessenen Puls defibrilliert werden soll, erfolgt durch Vergleich der Pulsfrequenz bzw. des Blutflusses mit Sollwerten. Das gemessene Geschwindigkeitsprofil des Blutes, gegebenenfalls in Kombination mit der Pulsfrequenz kann ausserdem zur Unterdrükung von Störungen, beispielsweise Bewegungsartefakten eingesetzt werden.

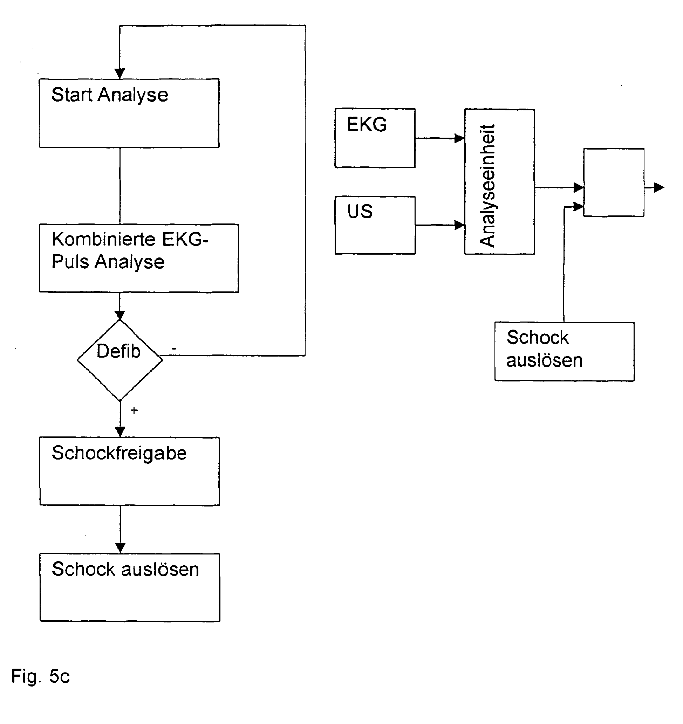

In Figur 5c ist ein Ausführungsbeispiel eines Analysezyklus gezeigt, bei welchem das den Blutfluss repräsentierende Hilfssignal gleichzeitig mit einem EKG-Signal in eine Analyse-Einheit gespiesen wird. Die kombinierte Analyse von EKG und Puls erlaubt die Verifikation der ermittelten Resultate. Da EKG und Puls zeitlich in Relation miteinander stehen, erlaubt die kombinierte Analyse die Eliminierung von Messfehlern.

Ansonsten funktioniert das Ausführungsbeipiel gemäss Figur 5c ähnlich wie Figuren 5a und 5b. Ein Defibrillationsstoss wird nur ausgelöst, wenn EKG als defibrillationswürdig gilt und die Flussmessung einen nicht ausreichenden Blutfluss anzeigt.

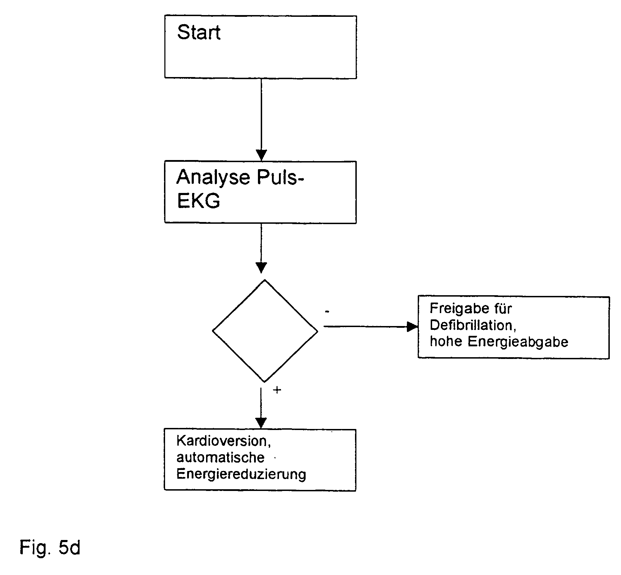

In Figur 5d ist ein Ausführungsbeispiel gezeigt, welches automatisch zwischen einer regulären Defibrillation und Defibrillation bei einer Kardioversion unterscheidet.

Aufgrund der Analyse von Puls und EKG wird entschieden, ob eine konventionelle Defibrillation notwendig ist. In diesem Fall wird ein Defibrillationsstromstoss mit einer hohen Energie abgegeben.

Im Fall einer Kardioversion (beispielsweise erkennbar durch zu hohe Pulsfrequenz) wird automatisch die Energie des Defibrillationsstromstosses reduziert.

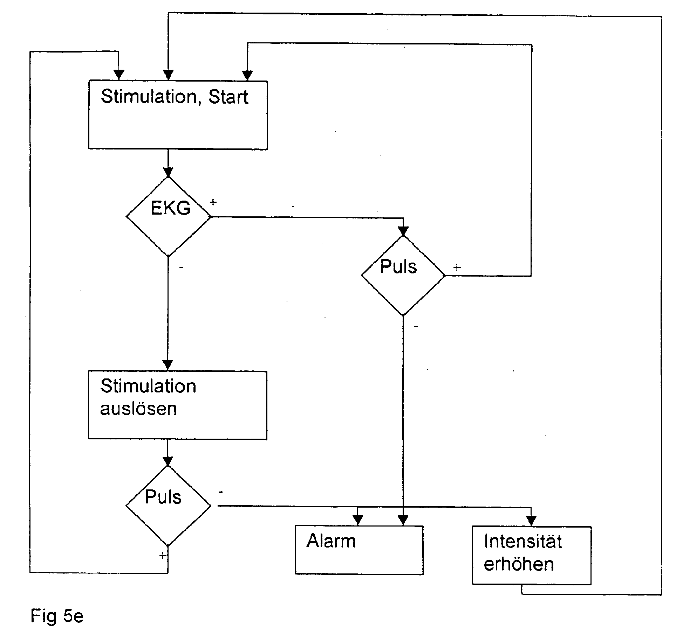

In Figur 5e schliesslich ist schematisch ein Analysezyklus gezeigt, bei welchem das Hilfssignal gemäss der Erfindung zur Überwachung und Steuerung eines temporären externen Herzschrittmachers eingesetzt wird.

Bei Vorhandensein eines ausreichenden EKG-Signals wird das Hilfssignal getestet und es wird entschieden, ob ausreichender Puls herrscht. Falls dies nicht der Fall ist, wird ein Alarm ausgelöst.

Wenn kein ausreichendes EKG ermittelt wird, wird eine externe Stimulation ausgelöst. Anschliessend wird mittels dem Hilfssignal der Puls gemessen. Falls nach der Stimulation immer noch kein Puls erkennbar ist, wird entweder Alarm ausgelöst oder gegebenenfalls eine Stimulation mit erhöhter Intensität durchgeführt.

In den verschiedenen Analysezyklen gemäss Figuren 5a bis 5e wird das Hilfssignal dazu verwendet, automatisch den Carotis-Puls zu ermitteln und zu überprüfen.

## Patentansprüche

1. Vorrichtung (3) zum Erzeugen eines Hilfssignals (H) zum Bestimmen des Zeitpunkts und/oder der Notwendigkeit für eine Defibrillation und/oder für eine Herzmassage bei Herzstörungen eines Säugetiers, insbesondere bei einem Menschen (1), welche Mittel (7) zum Erzeugen eines Hilfssignals (H) aufweist, wobei die Mittel (7) zum Erzeugen eines Hilfssignals (H) mit einem Defibrillator oder mit einer Anordnung zum Ausführen einer Herzmassage koppelbar sind, **gekennzeichnet durch** eine Anordnung (6) zum nicht-invasiven Messen des Wertes (W) des Blutflusses (B) in den Kopf des Säugetieres, welche in Wirkverbindung mit den Mitteln (7) zum Erzeugen eines Hilfssignals (H) steht.

2. Vorrichtung nach Anspruch 1, **dadurch gekennzeichnet, dass** die Vorrichtung eine Anordnung zum Erzeugen eines EKG-Signals aufweist.

3. Vorrichtung nach Anspruch 1 oder 2, **dadurch gekennzeichnet, dass** die Mittel (7) einen akustischen Signalgeber aufweisen.

4. Vorrichtung nach Anspruch 1 oder 2, **dadurch gekennzeichnet, dass** die Mittel (7) einen optischen Signalgeber aufweisen.

5. Vorrichtung nach einem der Ansprüche 1 bis 4, **dadurch gekennzeichnet, dass** die Anordnung (6) eine Messanordnung (10, 10') zum nicht-invasiven Messen des Blutflusses (B) ist, wobei die Messanordnung (10,10') eine Vielzahl von nebeneinander angeordneten Flussmesseinheiten (11, 11') aufweist, deren Ausgänge (12) mit einer Signalverarbeitungsanordnung (8, 13) gekoppelt sind.

6. Vorrichtung nach Anspruch 5, **dadurch gekennzeichnet, dass** die Anordnung (10) 4 bis 10 Flussmesseinheiten (11) aufweist.

7. Vorrichtung nach einem der Ansprüche 5 oder 6, **dadurch gekennzeichnet, dass** jede Flussmesseinheit (11, 11') einen Durchmesser von 0,5 bis 1 cm aufweist.

8. Vorrichtung nach einem der Ansprüche 5 bis 7, **dadurch gekennzeichnet, dass** die Flussmesseinheiten (11, 11') im continuous wave Verfahren arbeitende Dopplereffekt-Messeinheiten sind.

9. Vorrichtung nach einem der Ansprüche 5 bis 7, **dadurch gekennzeichnet, dass** die Flussmesseinheiten (11, 11') im gepulsten Verfahren arbeitende Dopplereffekt-Messeinheiten sind.

10. Vorrichtung nach einem der Ansprüche 5 bis 9, **dadurch gekennzeichnet, dass** die Flussmesseinheiten (11, 11') in einer Reihe nebeneinanderliegend angeordnet sind.

11. Vorrichtung nach einem der Ansprüche 5 bis 9, **dadurch gekennzeichnet, dass** die Flussmesseinheiten (11, 11') in zwei Reihen versetzt zueinander angeordnet sind.

## Claims

1. Device (3) for generating an auxiliary signal (H) for de termining the time and/or necessity of a defibrillation and/or a cardiac massage in the event of cardiac disturbances of a mammal, in particular in the case of a human (1), which has means (7) for generating an auxiliary signal (H), it being possible for the means (7) for generating an auxiliary signal (H) to be coupled to a defibrillator or to an arrangement for executing a cardiac massage, **characterized by** an arrangement (6) for non-invasive measurement of the value (W) of the blood flow (B) into the head of the mammal which is operationally connected to the means (7) for generating an auxiliary signal (H).

2. Device according to Claim 1, **characterized in that** the device has an arrangement for generating an ECG signal.

3. Device according to Claim 1 or 2, **characterized in that** the means (7) have an acoustic signal transmitter.

4. Device according to Claim 1 or 2, **characterized in that** the means (7) have an optical signal transmitter.

5. Device according to one of Claims 1 to 4, **characterized in that** the arrangement (6) is a measuring arrangement (10, 10') for non-invasive measurement of the blood flow (B), the measuring arrangement (10, 10') having a multiplicity of juxtaposed flow measuring units (11, 11') whose outputs (12) are coupled to a signal processing arrangement (8, 13).

6. Device according to Claim 5, **characterized in that** the arrangement (10) has 4 to 10 flow measuring units (11).

7. Device according to either of Claims 5 and 6, **characterized in that** each flow measuring unit (11, 11') has a diameter of 0.5 to 1 cm.

8. Device according to one of Claims 5 to 7, **characterized in that** the flow measuring units (11, 11') are Doppler effect measuring units operating using the continuous wave method.

9. Device according to one of Claims 5 to 7, **characterized in that** the flow measuring units (11, 11') are Doppler effect measuring units operating using the pulsed method.

10. Device according to one of Claims 5 to 9, **characterized in that** the flow measuring units (11, 11') are arranged juxtaposed in a row.

11. Device according to one of Claims 5 to 9, **characterized in that** the flow measuring units (11, 11') are arranged offset from one another in two rows.

## Revendications

1. Dispositif (3) de génération d'un signal auxiliaire (H) pour la détermination du moment et/ou de la nécessité d'une défibrillation et/ou d'un massage cardiaque en cas de défaillances cardiaques d'un mammifère, particulièrement d'un être humain (1), ce dispositif présentant des moyens (7) de génération d'un signal auxiliaire (H), les moyens (7) de génération d'un signal auxiliaire (H) pouvant être couplés à un défibrillateur ou à un dispositif de réalisation d'un massage cardiaque, **caractérisé par** un dispositif (6) de mesure non invasive de la valeur (W) du débit sanguin (B) dans la tête du mammifère, en liaison fonctionnelle avec les moyens (7) de génération d'un signal auxiliaire (H).

2. Dispositif selon la revendication 1, **caractérisé en ce que** le dispositif comporte un dispositif de génération d'un signal d'ECG.

3. Dispositif selon la revendication 1 ou 2, **caractérisé en ce que** les moyens (7) comportent un émetteur acoustique de signaux.

4. Dispositif selon la revendication 1 ou 2, **caractérisé en ce que** les moyens (7) comportent un émetteur optique de signaux.

5. Dispositif selon l'une quelconque des revendications 1 à 4, **caractérisé en ce que** le dispositif (6) est un dispositif de mesure (10, 10') pour la mesure non invasive du débit sanguin (B), ce dispositif de mesure (10, 10') comportant une multitude d'unités de mesure de débit juxtaposées (11, 11') dont les sorties (12) sont couplées à un dispositif de traitement de signaux (8, 13).

6. Dispositif selon la revendication 5, **caractérisé en ce que** le dispositif (10) comporte 4 à 10 unités de mesure de débit (11).

7. Dispositif selon l'une quelconque des revendications 5 ou 6, **caractérisé en ce que** chaque unité de mesure de débit (11, 11') a un diamètre allant de 0,5 à 1 cm.

8. Dispositif selon l'une quelconque des revendications 5 à 7, **caractérisé en ce que** les unités de mesure de débit (11, 11') sont des unités de mesure à effet Doppler fonctionnant suivant un procédé d'onde continue.

9. Dispositif selon l'une quelconque des revendications 5 à 7, **caractérisé en ce que** les unités de mesure de débit (11, 11') sont des unités de mesure à effet Doppler fonctionnant suivant un procédé à impulsions.

10. Dispositif selon l'une quelconque des revendications 5 à 9, **caractérisé en ce que** les unités de mesure de débit (11, 11') sont disposées de manière juxtaposée sur une rangée.

11. Dispositif selon l'une quelconque des revendications 5 à 9, **caractérisé en ce que** les unités de mesure de débit (11, 11') sont disposées de manière décalée les unes par rapport aux autres sur deux rangées.
